# FASCICULE DE BREVET EUROPEEN

(11) **EP 3 250 699 B1**
(45) Date de publication et mention de la délivrance du brevet: **28.11.2018**
(21) Numéro de dépôt: 16705239.8
(22) Date de dépôt: 26.01.2016
(51) Int. Cl.: C12P 7/64, C12P 13/06, C12P 13/14, C12N 1/12

(54) **PROCÉDÉ D'ENRICHISSEMENT DE LA BIOMASSE DE MICROALGUES DU GENRE TRAUSTOCHYTRIUM EN DHA ET EN ACIDES AMINÉS ARG ET GLU**
VERFAHREN ZUR ANREICHERUNG VON DHA, ARGININ UND GLUTAMINSÄURE IN DER BIOMASSE VON TRAUSTOCHYTRIUM MIKROALGEN IN DHA UND IN ARGININE UND GLUTAMINSÄURE
ENRICHMENT PROCESS OF THE THRAUSTOCHYTRIUM SPECIES MICROALGAE BIOMASS IN DHA AND IN ARGININE AND GLUTAMIC ACID

(30) Priorité: 27.01.2015 FR 1550598
(43) Date de publication de la demande: 06.12.2017
(73) Titulaire: Roquette Frères, 62136 Lestrem (FR)
(72) Inventeur: CAULIER, Bernard, 59273 Fretin (FR)
(74) Mandataire: Cabinet Plasseraud
(86) Numéro de dépôt international: PCT/FR2016/050159
(87) Numéro de publication internationale: WO 2016/120558

(56) Documents cités:
- WO-A1-01/54510
- WO-A2-2014/154787
- FR-A1- 3 001 736
- LI J ET AL: "Comparative metabolomics analysis of docosahexaenoic acid fermentation processes by Schizochytrium sp. under different oxygen availability conditions", OMICS A JOURNAL OF INTEGRATIVE BIOLOGY, MARY ANN LIEBERT, NEW YORK, NY, US, vol. 17, no. 5, 1 mai 2013 (2013-05-01), pages 269-281, XP002728860, ISSN: 1536-2310, DOI: 10.1089/OMI.2012.0088 [extrait le 2013-04-15]
- REN LU-JING ET AL: "Regulation of docosahexaenoic acid production bySchizochytriumsp.: effect of nitrogen addition", BIOPROCESS AND BIOSYSTEMS ENGINEERING, SPRINGER, DE, vol. 37, no. 5, 21 septembre 2013 (2013-09-21), pages 865-872, XP035315944, ISSN: 1615-7591, DOI: 10.1007/S00449-013-1057-5 [extrait le 2013-09-21]
- ANITA N JAKOBSEN ET AL: "Accumulation of docosahexaenoic acid-rich lipid in thraustochytrid Aurantiochytrium sp. strain T66: effects of N and P starvation and O2 limitation", APPLIED MICROBIOLOGY AND BIOTECHNOLOGY, SPRINGER, BERLIN, DE, vol. 80, no. 2, 17 juin 2008 (2008-06-17), pages 297-306, XP019623690, ISSN: 1432-0614

## Description

La présente invention se rapporte à un nouveau procédé fermentaire d'enrichissement de la biomasse de microalgues du genre *Thraustochytrium,* plus particulièrement *Schizochytrium sp ou Schizochytrium mangrovei,* en acide docosahexaénoïque (ou DHA) et en acides aminés arginine et acide glutamique, ainsi qu'un procédé de production de l'huile extraite de cette biomasse de microalgues.

### Le domaine technique des lipides

Les lipides constituent une des trois grandes familles de macronutriments avec les protéines et les glucides.

Parmi les lipides, on distingue notamment les triglycérides et les phospholipides :
- Les triglycérides (également appelés triacylglycérols ou triacylglycérides ou TAG) sont des glycérides dans lesquels les trois groupements hydroxyles du glycérol sont estérifiés par des acides gras. Ils sont le constituant principal de l'huile végétale et des graisses animales.

Les triglycérides représentent environ 95 % des lipides alimentaires ingérés par l'Homme. Dans l'organisme, ils sont présents principalement dans les tissus adipeux et constituent la forme principale de stockage de l'énergie.
- Les phospholipides sont des lipides amphiphiles, c'est-à-dire constitués d'une «tête» polaire (hydrophile) et de deux « queues » aliphatiques (hydrophobes).

Les phospholipides sont des lipides de structure car ils sont des constituants des membranes cellulaires dont ils assurent entre autre la fluidité.

Triglycérides et phospholipides sont composés majoritairement d'acides gras qui sont à la fois apportés par l'alimentation et, pour certains d'entre eux, synthétisés par l'organisme.

La classification biochimique (basée sur le nombre de doubles liaisons contenues dans la molécule d'acide gras) distingue les acides gras saturés (AGS), les acides gras monoinsaturés (AGMI) et les acides gras polyinsaturés (AGPI).

Du point de vue physiologique, on distingue :
- les acides gras indispensables nécessaires au développement et au bon fonctionnement du corps humain, mais que notre corps ne sait pas fabriquer ;
- les acides gras dit « conditionnellement » indispensables, essentiels pour la croissance normale et les fonctions physiologiques des cellules mais qui peuvent être fabriqués à partir de leur précurseur s'il est apporté par l'alimentation. Ils sont donc rigoureusement requis si leur précurseur indispensable est absent.
- les acides gras non indispensables.

L'ensemble des acides gras indispensables et « conditionnellement » indispensables constituent les acides gras essentiels.

Les autres acides gras sont dits non essentiels.

Parmi les acides gras non indispensables, on trouve notamment :
- l'acide eicosapentaénoïque (EPA) de la famille des acides gras oméga 3,
- l'acide oléique, l'acide gras monoinsaturé majoritaire dans notre alimentation, et l'acide palmitoléique,
- les acides gras saturés, tels l'acide laurique, l'acide myristique ou l'acide palmitique.

Plus particulièrement, les acides gras polyinsaturés sont classés en fonction de la position de la première double liaison, à partir de la fonction méthyle finale.

Ainsi, dans la nomenclature, pour omega «x» ou « nx », «x» correspond à la position de la première insaturation.

On distingue deux grandes familles d'acides gras essentiels : les acides gras oméga 6 (ou AGPI n-6), dont le précurseur et le représentant majeur est l'acide linoléique (LA) et les acides gras oméga 3 (ou AGPI n-3) dont le précurseur est l'acide alpha-linolénique (ALA).

La majorité des acides gras polyinsaturés d'intérêt biologique appartient à la famille des omega 6 (acide arachidonique ou ARA) ou omega 3 (acide eicosapentaénoïque ou EPA, acide docosahexaénoïque ou DHA).

En outre, dans la nomenclature, on définit également le nombre de carbone constituant la chaîne ; ainsi l'EPA est décrit comme C20:5 et le DHA comme C22:6.

Le « 5 » et « 6 » correspondent ainsi au nombre d'insaturations de la chaîne carbonée présentés respectivement par l'EPA et par le DHA.

Le DHA, de la famille des acides gras oméga 3, est un acide gras que l'organisme sait synthétiser à partir de l'acide alpha-linolénique, ou qui est apporté par la consommation de poissons gras (thon, saumon, hareng...).

Le DHA joue un rôle important dans la structure des membranes et dans le développement et le fonctionnement du cerveau et de la rétine.

Les huiles de poisson sont utilisées principalement comme source d'acides gras de type omega 3, tels le DHA et l'EPA, mais on les trouve également dans les huiles de microalgues à partir desquelles on les extrait soit en mélange, soit séparément, comme c'est le cas par exemple des huiles issues de certaines souches sélectionnées, telles que celles du genre *Schizochytrium,* qui ne contiennent que des traces d'EPA mais de fortes teneurs en DHA.

### Le domaine technique des peptides et acides aminés

Les peptides et acides aminés sont classiquement valorisés comme agents fonctionnels ou compléments alimentaires dans de nombreux domaines.

Dans le cadre de l'apport en acides aminés d'intérêt, il peut être en effet avantageux de disposer de sources de peptides riches en arginine et en acide glutamique.

L'arginine est un acide aminé qui présente de nombreuses fonctions dans le règne animal.

L'arginine peut être dégradée et ainsi servir de source d'énergie, de carbone et d'azote à la cellule qui l'assimile.

Chez divers animaux, dont les mammifères, l'arginine est décomposée en ornithine et en urée. Cette dernière est une molécule azotée qui peut être éliminée (par excrétion dans les urines) de manière à réguler la quantité de composés azotés présente dans les cellules des organismes animaux.

L'arginine permet la synthèse du monoxyde d'azote (NO) par la NO synthétase, intervenant ainsi dans la vasodilatation des artères, ce qui réduit la rigidité des vaisseaux sanguins, augmente le flux sanguin et améliore ainsi le fonctionnement des vaisseaux sanguins.

Les compléments alimentaires qui contiennent l'arginine sont recommandés pour promouvoir la santé du coeur, la fonction vasculaire, pour prévenir « l'agrégation des plaquettes » (risque de formation de caillots sanguins) et pour abaisser la pression artérielle.

L'implication de l'arginine dans la cicatrisation des plaies est liée à son rôle dans la formation de la proline, un autre acide aminé important pour la synthèse de collagène.

L'arginine est enfin un composant fréquemment utilisé, notamment par les sportifs, dans les boissons énergisantes.

L'acide glutamique quant à lui n'est pas seulement l'une des briques élémentaires utilisées pour la synthèse des protéines, mais est aussi le neurotransmetteur excitateur le plus répandu dans le système nerveux central (encéphale + moelle épinière) et est un précurseur du GABA dans les neurones GABAergiques.

Sous le code de «E620», le glutamate est utilisé comme exhausteur de goût des aliments. Il est additionné aux préparations alimentaires pour renforcer leur goût.

Outre le glutamate, le Codex Alimentarius a reconnu aussi comme exhausteurs de goût ses sels de sodium (E621), de potassium (E622), calcium (E623), ammonium (E624) et magnésium (E625).

Le glutamate (ou ses sels) est souvent présent dans les plats tout prêts (soupes, sauces, chips, plats cuisinés). Il est aussi couramment utilisé en cuisine asiatique.

Il est actuellement fréquemment utilisé en combinaison avec des arômes dans les apéritifs (goût bacon, goût fromage). Cela permet de rehausser le goût de bacon, du fromage, etc. Il est rare de trouver un apéritif qui n'en contienne pas.

On en trouve aussi dans certaines capsules de médicaments mais pas pour ses fonctions gustatives.

Enfin, il est le composant majoritaire des auxiliaires de cuisine (bouillons cubes, fonds de sauces, sauces, etc.).

### Production de lipides, notamment d'acides gras, par les microalgues

La culture des microalgues du genre *Schizochytrium* est réalisée classiquement dans des fermenteurs (conditions hétérotrophiques : à l'obscurité en présence d'une source carbonée).

Il est à noter que l'exploitation rentable de ces microalgues nécessite généralement la maîtrise des conditions de fermentation.

Pour parvenir à ce résultat, de premiers procédés de fermentation permettant d'obtenir de hautes densités cellulaires (acronyme anglais : HCD pour *High-Cell-Density*) ont été ainsi beaucoup travaillés, de manière à obtenir des rendements et productivités maximales en lipides.

L'objectif de ces cultures HCD était l'obtention de la concentration la plus élevée possible des lipides souhaités dans le laps de temps le plus court.

Cependant, il est vite apparu aux spécialistes du domaine qu'il faut par exemple soumettre les microalgues à un stress nutritionnel qui limite leur croissance lorsqu'on souhaite leur faire produire d'importantes réserves lipidiques.

Il est donc classiquement procédé au découplage croissance / production dans les procédés fermentaires.

Par exemple, pour favoriser l'accumulation d'acides gras polyinsaturés (ici l'acide docosahexaénoïque ou DHA), la demande de brevet WO 01/54510 recommande de dissocier la croissance cellulaire et la production d'acides gras polyinsaturés.

Il est plus particulièrement revendiqué un procédé pour la production de lipides microbiens, comprenant les étapes consistant à :
(a) effectuer une fermentation d'un milieu comprenant des microorganismes, une source de carbone et une source nutritive limitante et en assurant des conditions suffisantes pour maintenir un taux d'oxygène dissous d'au moins environ 4 % de la saturation dans ledit milieu de fermentation pour augmenter la biomasse ;
(b) puis fournir des conditions suffisantes pour maintenir un taux d'oxygène dissous approximativement égal ou inférieur à 1 % de la saturation dans ledit milieu de fermentation et fournir des conditions suffisantes pour permettre auxdits microorganismes de produire lesdits lipides ;
(c) et recueillir lesdits lipides microbiens, dans lequel au moins environ 15 % desdits lipides microbiens sont constitués de lipides polyinsaturés ;
   et dans lequel une densité de biomasse d'au moins environ 100 g/l est obtenue au cours de la fermentation.

Chez la microalgue *Schizochytrium sp* souche ATCC 20888, il est ainsi plus particulièrement procédé à une première phase de croissance en présence d'une source carbonée et d'une source azotée mais sans limitation en oxygène, de manière à favoriser l'obtention d'une haute densité cellulaire puis, dans une deuxième phase, arrêter la fourniture d'azote et ralentir progressivement l'apport en oxygène (gestion de la pression en oxygène dissous ou pO₂ de 10 %, à 4 %, puis 0,5 %), afin de stresser la microalgue, ralentir sa croissance et enclencher la production des acides gras d'intérêt.

Li J. et al (OMICS A Journal of Integrative Biology, vol. 17(5), 2013, pages 269-281) divulgue un procédé d' enrichissement d'une biomasse de microalgues de genre Schizochytrium en acides aminés. Chez la microalgue *Crypthecodinium cohnii,* la teneur la plus élevée en DHA est obtenue à faible concentration en glucose (de l'ordre de 5 g/l), et ainsi à faible taux de croissance (Jiang and Chen, 2000, Process Biochem., 35(10), 1205-1209).

De ce fait, dans les cas où la formation des produits n'est pas corrélée avec une croissance cellulaire élevée, il est enseigné qu'il est judicieux de maîtriser le taux de croissance cellulaire.

En général, l'homme du métier choisit de contrôler la croissance des microalgues par la maîtrise des conditions de fermentation (Température, pH...), ou par l'alimentation régulée en composants nutritionnels du milieu de fermentation (conditions semi continues dites «fed-batch»).

S'il choisit de contrôler la croissance des microalgues en hétérotrophie par l'apport en sources carbonées, l'homme du métier choisit généralement d'adapter la source carbonée (glucose pur, acétate, éthanol...) à la microalgue (*C. cohnii, Euglena gracilis...*) en fonction du métabolite produit (par exemple un acide gras polyinsaturé de type DHA).

La température peut également être un paramètre clef. Il a par exemple été rapporté que la synthèse d'acides gras polyinsaturés chez certaines espèces de microalgues, tel que l'EPA par *Chlorella minutissima,* est favorisée à une plus basse température que celle requise pour la croissance optimale de ladite microalgue.

Pour optimiser la production en triglycérides, l'homme du métier est également amené à optimiser le flux carboné vers la production d'huile, en agissant sur l'environnement nutritionnel du milieu de fermentation.

Il est ainsi connu que l'accumulation en huile se produit lors d'un apport carboné suffisant, mais dans des conditions de carence en azote.

Le rapport C/N est donc ici déterminant, et il est admis que les meilleurs résultats sont obtenus en agissant directement sur la teneur en azote, la teneur en glucose n'étant pas limitante.

Pour optimiser la production en huile, il est donc primordial pour l'homme du métier de contrôler le flux carboné en le déviant vers la production d'huile, au détriment de la production des protéines ; le flux carboné est redistribué et s'accumule en substances de réserve lipidiques quand les microalgues sont placées en milieu carencé en azote.

### Production de protéines par les microalgues

Comme il est détaillé ci-avant, pour optimiser la production en triglycérides, l'homme du métier est amené à optimiser le flux carboné vers la production d'huile, en agissant sur l'environnement nutritionnel du milieu de fermentation.

Dans une étude réalisée chez une microalgue de type *Chlorella,* il a été noté qu'une carence en azote affecte la croissance cellulaire, ce qui résulte en un taux de croissance diminué de 30 % par rapport au taux de croissance normal de la microalgue (Xiong et al., Plant Physiology, 2010, 154, pp1001-1011).

Pour expliquer ce résultat, Xiong et al, dans l'article cité supra, démontrent en effet que, si l'on divise la biomasse de *Chlorella* en ses 5 composants principaux, notamment hydrates de carbone, lipides, protéines, ADN et ARN (représentant 85 % de sa matière sèche), le rapport C/N n'a aucun impact sur la teneur en ADN, ARN et hydrates de carbone, mais il devient prééminent pour le contenu en protéines et en lipides.

C'est ainsi que les cellules de Chlorelles cultivées avec un rapport C/N faible contiennent 25,8 % de protéines et 25,23 % en lipides, tandis qu'un rapport C/N élevé permet la synthèse de 53,8 % de lipides et 10,5 % de protéines.

Pour optimiser la production en protéines, il est donc primordial pour l'homme du métier de contrôler le flux carboné en le déviant vers la production des protéines au détriment de la production des lipides ; le flux carboné est redistribué et s'accumule en substances de réserve protéiques quand les microalgues sont placées en milieu non carencé en azote.

Fort de cet enseignement, pour la production de biomasses riches en protéines et donc en acides aminés qui les constituent, l'homme du métier est donc amené à travailler les conditions de fermentation en favorisant plutôt un rapport C/N faible, et ainsi :
- réaliser un apport important en source d'azote au milieu de fermentation, tout en maintenant constant la charge en source carbonée qui sera convertie en protéines et
- stimuler la croissance de la microalgue.

### RESUME DE L'INVENTION

La présente invention est relative à un procédé de production d'une biomasse de microalgues du genre *Thraustochytrium* dont la fraction lipidique est riche en DHA et dont la teneur en acides aminés arginine et acide glutamique sur acides aminés totaux est élevée.

Ce procédé repose sur le contrôle du taux de croissance de la microalgue, ce contrôle s'exerçant de manière à le réduire à son minimum, tout en maintenant ou en introduisant en continu une source d'azote dans le milieu de fermentation.

Ce résultat peut par exemple être obtenu par la réduction ou l'épuisement en oligoéléments du milieu de fermentation ou par la limitation du transfert en O₂.

Dans un mode préférentiel de réalisation du procédé conforme à l'invention, il est ainsi choisi de limiter le taux de croissance de la microalgue en limitant l'apport d'oxygène.

Au sens de l'invention, la limitation du taux de croissance s'apprécie par le rapport entre le taux de croissance réel de la microalgue (µ) en regard de son taux de croissance optimal (µmax), où «µ» est la vitesse de croissance exprimée en g de biomasse formée par g de biomasse et par heure, soit (h⁻¹).

Plus particulièrement, le procédé de l'invention est un procédé d'enrichissement d'une biomasse de microalgues du genre *Thraustochytrium* en DHA et en acides aminés arginine et acide glutamique, caractérisé en ce qu'il comprend une étape consistant à maintenir ou ajouter une source d'azote dans le milieu de fermentation dès que la valeur du rapport des taux de croissance µ/µmax des microalgues devient inférieur à 0,2.

De préférence, les microalgues sont du genre *Schizochytrium sp ou Schizochytrium mangrovei.*

De manière plus spécifique, les microalgues peuvent être une souche sélectionnée parmi les souches CNCM I-4469 et CNCM I-4702 déposées à la Collection Nationale de Cultures de Microorganismes de l'Institut Pasteur respectivement le 14 avril 2011 et le 22 novembre 2012.

Facultativement, le procédé peut comprendre en outre la récolte de la biomasse, éventuellement la préparation d'un extrait ou lysat cellulaire à partir de cette biomasse, puis facultativement l'extraction d'une huile brute riche en DHA et en acides aminés arginine et acide glutamique.

Le procédé selon la présente invention peut être caractérisé en ce que la biomasse obtenue comprend :
- au minimum 45 % de DHA en poids d'acides gras totaux ; et
- au minimum 10% d'arginine et au moins 25 % d'acide glutamique en poids sur acides aminés totaux, de préférence au minimum 15% d'arginine et au moins 40 % d'acide glutamique en poids sur acides aminés totaux.

### DESCRIPTION DETAILLEE DE L'INVENTION

Dans le cadre de l'invention, la société Demanderesse a choisi d'explorer une voie originale d'optimisation de la production en DHA et en acides aminés arginine et acide glutamique en proposant une nouvelle conduite de fermentation.

La société Demanderesse a ainsi trouvé, ce qui va à l'encontre des préjugés techniques en la matière, qu'il est possible de produire par fermentation des biomasses de microalgues :
- riches en lipides (plus de 25 % en poids sec de biomasse, de préférence au minimum 30 %) dont l'acide gras prépondérant est l'acide docosahexaénoïque (DHA), et
- riches en acides aminés arginine et acide glutamique (plus de 35 %, en poids des acides aminés totaux, de préférence au minimum 55 %),
sans qu'il ne soit indispensable, comme décrit dans l'état de l'art, de maximiser le rapport C/N (carbone consommé sur azote consommé mole/mole).

La société Demanderesse a ainsi trouvé que l'on peut modifier la composition en lipides et en acides aminés de la biomasse produite par fermentation, grâce au maintien, non conventionnel pour une production de lipides, de l'alimentation en azote tout au long de la fermentation même lorsque le taux de croissance µ/µmax est inférieur à 0,2.

En effet, la société Demanderesse a compris que lorsque le ratio µ/µmax devient inférieur à 0,2, suite à une limitation en un substrat nutritif autre que les substrats azotés ou carbonés, il est possible de dévier les productions métaboliques vers la production d'acides aminés arginine et acide glutamique, tout en conservant une production de DHA importante.

Dans un mode de réalisation, la limitation permettant de diminuer le taux de croissance peut être la limitation de l'apport en oxygène (OTR, vitesse de transfert de l'oxygène).

Notamment, l'OTR pendant la phase de fermentation est de préférence de 30 à 35 mmoles /L/h.

La limitation de croissance peut également être induite par l'épuisement en oligoéléments ou minéraux, de préférence choisis parmi le phosphate, le magnésium ou le potassium.

Plus particulièrement la société Demanderesse a trouvé qu'il faut réaliser un apport en azote, préférentiellement sous forme d'ammoniaque (utilisé par exemple en régulation de pH), ou qu'il faut maintenir l'apport en azote, jusqu'à la fin de la culture, dès lors que le µ vaut moins de 20 % de µmax.

Dans un mode de réalisation préféré, l'apport en azote initial est complété par la régulation du pH, l'azote consommé étant ainsi compensé par celui de la régulation du pH. Cela permet d'obtenir un ratio C/N (carbone consommé sur azote consommé mole/mole) en fin de culture inférieur à 20, par exemple compris entre 10 et 15, et de préférence d'environ 15.

Les souches à utiliser dans les méthodes de la présente invention sont du genre *Thraustochytrium,* plus particulièrement *Schizochytrium mangrovei* ou *Schizochytrium sp.* De telles souches sont connues de l'homme du métier.

La société Demanderesse a identifié au cours de ses recherches plusieurs souches de microalgues productrices de DHA de grand intérêt. Notamment, la société Demanderesse est tout particulièrement intéressée par deux souches qu'elle a identifiées.

La première souche est une souche de Schizochytrium sp., déposée en France le 14 avril 2011 auprès de la Collection Nationale de Cultures de Microorganismes de l'institut Pasteur (CNCM), 25 rue de Docteur Roux, 75724 Paris Cedex 15, France, sous le numéro I-4469 mais également en Chine auprès du CHINA CENTER FOR TYPE CULTURE COLLECTION (CCTCC) de l'université de Wuhan, Wuhan 430072, P.R. China sous le numéro M 209118. Cette souche produit principalement du DHA et en moindre mesure de l'acide palmitique et de l'acide palmitoléïque. Elle a été caractérisée par séquençage partiel du gène codant pour l'ARN 18S (SEQ ID No 1): ce qui a permis de l'identifier comme étant une souche du type *Schizochytrium* sp. Cette souche sera désignée «CNCM 1-4469» ultérieurement dans la présente demande.

Par ailleurs, la deuxième souche est une souche de *Schizochytrium mangrovei.* Elle produit du DHA et d'acide palmitique en proportion relativement égale. Elle a été déposée par la société Demanderesse en France le 22 novembre 2012 auprès de la Collection Nationale de Cultures de Microorganismes de l'Institut Pasteur (CNCM), 25 rue de Docteur Roux, 75724 Paris Cedex 15, France, sous le numéro CNCM I-4702. Elle a été caractérisée par séquençage des gènes codant pour l'ARNr 18 S (SEQ ID No 2): ce qui a permis de l'identifier comme étant une souche du type *Schizochytrium mangrovei.* Cette souche sera désignée «CNCM I-4702» ultérieurement dans la présente demande.

Par ailleurs, les procédés fermentaires selon la présente invention sont mis en oeuvre dans des conditions de culture hétérotrophiques. Ces conditions adaptées aux microalgues considérées ainsi que les milieux de culture sont bien connus de l'homme du métier.

La source carbonée nécessaire à la croissance de la microalgue est préférentiellement du glucose.

De préférence, l'apport en glucose est tel que la concentration en glucose pendant la fermentation est maintenue à une concentration de 20 g/L ou plus. En fin de fermentation, la concentration en glucose est d'au moins 5 g/L.

La source d'azote peut être des extraits de levure, de l'urée, du glutamate de sodium, du sulfate d'ammonium, de l'ammoniaque en régulation de pH, pris seuls ou en combinaison.

Généralement, l'étape de culture comprend une étape de préculture, pour revivifier la souche, puis une étape de culture ou de fermentation proprement dite. Cette dernière étape correspond à l'étape de production des lipides d'intérêt, en particulier de DHA.

De préférence, le pH est régulé pendant la fermentation à un pH compris entre 5 et 7, de préférence environ 6.

De préférence, la température lors de la fermentation est de 26-30 °C, de préférence environ 28 °C.

La durée de fermentation est de préférence d'au moins 50 heures, de préférence entre 65 et 90 heures, de manière encore plus préférée entre 70 et 85 heures.

Le procédé de fermentation selon la présente invention permet (ou est réalisé de manière à obtenir) d'obtenir une biomasse comprenant au minimum 45 % de DHA en poids d'acides gras totaux. En outre, le procédé garantit un taux de lipides en poids par rapport à la biomasse d'au moins 25 %. Ainsi, la biomasse est bien enrichie en DHA.

Par ailleurs, le procédé de fermentation selon la présente invention permet (ou est réalisé de manière à obtenir) d'obtenir une biomasse comprenant au minimum 40 % de protéines en poids par rapport à la biomasse. En outre, la proportion d'acide glutamique par rapport aux acides aminés totaux est d'au moins de 25 %. Celle en arginine est d'au moins de 10%.

Pour la souche CNCM I-4702, les résultats obtenus avec le procédé de fermentation selon l'invention sont une biomasse comprenant environ 47 % de DHA en poids d'acides gras totaux avec un taux de lipides en poids par rapport à la biomasse d'environ 35 %, et environ 53 % de protéines avec une proportion d'acide glutamique d'environ 40 % et d'arginine d'environ 16%.

Pour la souche CNCM I-4469, les résultats obtenus avec le procédé de fermentation selon l'invention sont une biomasse comprenant environ 52 % de DHA en poids d'acides gras totaux avec un taux de lipides en poids par rapport à la biomasse d'environ 26 %, et environ 43 % de protéines avec une proportion d'acide glutamique d'environ 26 % et d'arginine d'environ 10 %.

Lorsqu'il est fait référence à un pourcentage en poids, il est entendu en poids sec.

Outre la biomasse, la présente invention concerne également un extrait ou lysat cellulaire préparé à partir de cette biomasse. En particulier, cet extrait ou lysat est préparé à partir de la biomasse récupérée après fermentation. Cet extrait ou lysat riche en DHA et en acides aminés arginine et acide glutamique.

La rupture des cellules pour l'extraction du contenu lipidique peut être effectuée par différentes voies parmi lesquelles les voies mécanique, chimique, enzymatique.

Par la suite, une huile peut être extraite du lysat cellulaire.

Ainsi, la méthode de production de lipides d'intérêt, de préférence DHA, et d'acides aminés arginine et acide glutamique, comprend le procédé fermentaire selon la présente invention, la récolte de la biomasse, la préparation d'un extrait ou lysat cellulaire et extraction d'une huile brute comprenant les lipides d'intérêt, de préférence DHA, et facultativement d'acides aminés arginine et acide glutamique.

Par « environ » est entendu la valeur + ou - 10 % de celle-ci, de préférence + ou - 5 % de celle-ci.

L'invention sera mieux comprise à l'aide des exemples qui suivent, lesquels se veulent illustratifs et non limitatifs.

### EXEMPLES

### Exemple 1 : Conditions de cultures de la souche CNCM I-4702

Le protocole comprend une préculture pour un ensemencement du fermenteur à 0,1 g/L de biomasse *pour la souche Schizochytrium mangrovei* CNCM I-4702.

### Préculture

La préculture (100 ml de milieu) en erlens de 500 ml à chicanes dure 24 h à 28°C. L'ensemble des composants du milieu est stérilisé par filtration.

**Tableau I**

| Milieu de préculture | % (g/g) |
|---|---|
| Glucose anhydre | 3 |
| Extrait de levure | 0,4 |
| Glutamate de sodium mono-sodique | 6,42 |
| NaCl | 1,25 |
| MgSO₄ 7(H₂O) | 0,4 |
| KCl | 0,05 |
| CaCl₂ 2(H₂O) | 0,01 |
| NaHCO₃ | 0,05 |
| KH2PO₄ | 0,4 |
| Vitamines solution mère B1, B6, B12 | 0,1 |
| Oligo-éléments solution mère | 0,8 |

### La culture

Le milieu est stérilisé en 3 parties.

Le glucose est stérilisé avec le KH2PO₄ pour un ajout juste avant T₀.

Le reste des sels est stérilisé en fermenteur avec 0,75 ml /L de Clearol FBA 3107. Les oligo-éléments et vitamines sont stérilisés par filtration.

Le volume à T₀ représente 75 % du volume final. Le pH est ajusté à T₀ par de l'ammoniaque puis il est régulé à 6 toujours à l'ammoniaque.

**Tableau II**

| Milieu de culture | % (P/P) |
|---|---|
| *KH2PO4* | 0,80 |
| *(NH4)2SO4* | 0,33 |
| *Na2SO4* | 0,67 |
| *NaCl* | 0,27 |
| *Ca Cl2 2 (H2O)* | 0,03 |
| *Mg SO4 7(H2O)* | 1,00 |
| *Glucose anhydre* | 6,00 |
| *Vitamines solution mère B1, B6, B12* | 0,20 |
| *Oligo-éléments solution mère* | 0,27 |

Un Fed batch de glucose (concentration : 500 g/l) est apporté en continu à partir de T₀ à rythme constant (à adapter d'après calculs) pour ne pas être à une concentration inférieure à 20 g/L. Au final, on épuisera le glucose sans descendre sous 5 g/L lors de l'arrêt de la fermentation.

La culture est conduite à 28 °C et dure de 70 à 85 heures avec un OTR (oxygen uptake rate ou vitesse de transfert de l'oxygène) fixe et constant de 20 à 30 mmoles d'O₂/L/h

### Solutions Mères

| vitamines | g/L |
|---|---|
| B1 | 45 |
| B6 | 45 |
| B12 | 0.25 |

| Oligo-éléments | g/L |
|---|---|
| MnCl2 2h20 | 8.60 |
| CoCl2 6H2O | 0.2 |
| NiSO4 6H2O | 7.50 |
| Na2MoO4 2H2O | 0.15 |
| ZnSO4 7H2O | 5.70 |
| Cu So4 5h2O | 6.50 |
| FeSO4 7 H2O | 32.00 |
| Acétate de Zinc | 0.01 |
| EDTA | Mis à pH > 3 |

Deux conditions de fermentation sont mises en oeuvre :
- En témoin : conditions dites « standard », dans lesquelles on maximise le ratio C/N (carbone consommé sur azote consommé) de manière à produire essentiellement des lipides en interrompant la fourniture d'azote mais pas celle du substrat carboné, ce sans limitation en O₂. Ces conditions sont donc carencées en azote.
   La suppression de la fourniture en azote s'effectue quand un ou plusieurs sels sont épuisés. La croissance proprement dite est alors impossible ou très limitée : la vitesse de multiplication cellulaire chute au profit de l'enrichissement en lipides des cellules présentes. La masse globale des cellules augmente mais le nombre de cellules évolue peu car le taux de croissance chute.
- Selon l'invention : Conditions permettant de produire des lipides riches en DHA avec des acides aminés riches en arginine et acide glutamique en limitant la vitesse de croissance par la limitation du transfert en O₂ de sorte que µ chute à µ/µmax < 0,2 rapidement, tout en maintenant la fourniture du glucose et de l'azote préférentiellement grâce à la régulation de pH à l'ammoniaque.

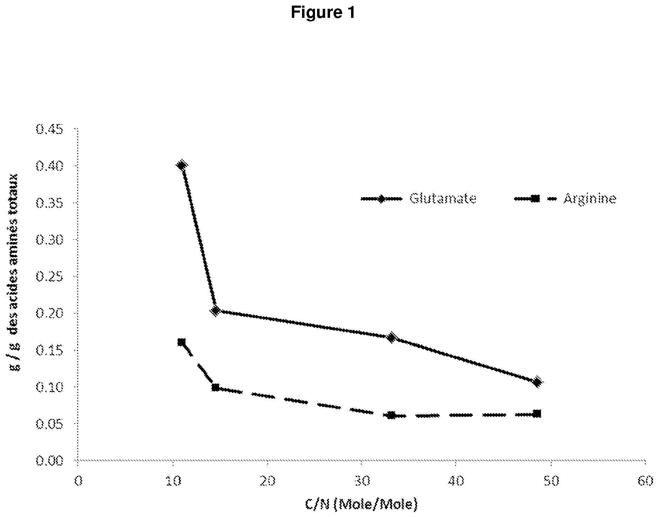

La figure 1 présente l'évolution de la proportion en arginine et acide glutamique parmi les acides aminés en fonction du C/N calculé en fin de culture.

Il apparait que le procédé favorise la production d'acides aminés arginine et acide glutamique dès lors que le rapport C/N est inférieur à 15 (# µ/µmax < 0,2).

Le tableau III ci-dessous traduit, pour la souche CNCM I-4702, la composition en acides gras et acides aminés de la biomasse produite selon les conditions opératoires « classique » et conforme à l'invention.

**Tableau III**

| | | **Culture classique** | **Utilisation du procédé de l'invention** |
|---|---|---|---|
| Lipides sur Biomasse | (g/g) | 0,60 | 0,35 |
| **Protéines sur Biomasse selon N 6.25** | **(g/g)** | **0,12** | **0,53** |
| **DHA / Acides gras** | **(g/g)** | **0,24** | **0,47** |
| | | | |
| Acide Aspartique sur Σ AAT | (g/g) | 0,12 | 0,05 |
| Thréonine sur Σ AAT | (g/g) | 0,06 | 0,03 |
| Serine sur Σ AAT | (g/g) | 0,06 | 0,03 |
| **Acide Glutamique sur Σ AAT** | **(g/g)** | **0,11** | **0,40** |
| Glycine sur Σ AAT | (g/g) | 0,05 | 0,03 |
| Alanine sur Σ AAT | (g/g) | 0,07 | 0,04 |
| Valine sur Σ AAT | (g/g) | 0,06 | 0,03 |
| Isoleucine sur Σ AAT | (g/g) | 0,05 | 0,03 |
| Leucine sur Σ AAT | (g/g) | 0,08 | 0,04 |
| Tyrosine sur Σ AAT | (g/g) | 0,04 | 0,02 |
| Phénylalanine sur Σ AAT | (g/g) | 0,04 | 0,03 |
| Lysine sur Σ AAT | (g/g) | 0,07 | 0,04 |
| Histidine sur Σ AAT | (g/g) | 0,02 | 0,01 |
| **Arginine sur Σ AAT** | **(g/g)** | **0,06** | **0,16** |
| Proline sur Σ AAT | (g/g) | 0,05 | 0,03 |
| Cystine sur Σ AAT | (g/g) | 0,02 | 0,01 |
| Méthionine sur Σ AAT | (g/g) | 0,03 | 0,02 |
| Tryptophane sur Σ AAT | (g/g) | 0,02 | 0,01 |

La proportion d'acide glutamique sur la somme des acides aminés est multipliée par 3.75 et la proportion d'arginine sur la somme des acides aminés est multipliée par 2.75.

La composition en lipide est réduite mais la teneur en DHA des acides gras est presque multipliée par deux.

### Exemple 2 : Conditions de cultures de la souche CNCM I-4469

Les conditions de culture de cette microalgue sont les mêmes que celles de l'exemple 1 (à l'exception du taux d'inoculum choisi en préculture, de l'ordre de 5 g/l pour *Schizochytrium* sp.).

Selon, deux conditions de culture menées de façon « classiques » et selon l'invention.

Le tableau IV ci-dessous traduit la composition en acides gras et acides aminés de la biomasse produite selon les conditions opératoires « classique » et conforme à l'invention.

**Tableau IV**

| | | **Culture classique** | **Utilisation du procédé de l'invention** |
|---|---|---|---|
| Lipides sur Biomasse (g/g) | | 0,46 | 0,26 |
| **Protéines sur Biomasse selon N 6.25** | **(g/g)** | **0,21** | **0,43** |
| **DHA / Acides gras (g/g)** | | **0,42** | **0,52** |
| | | | |
| Acide Aspartique sur Σ AAT | (g/g) | 0,12 | 0,08 |
| Thréonine sur Σ AAT | (g/g) | 0,05 | 0,04 |
| Serine sur Σ AAT | (g/g) | 0,05 | 0,04 |
| **Acide Glutamique sur Σ AAT** | **(g/g)** | **0,15** | **0,26** |
| Glycine sur Σ AAT | (g/g) | 0,05 | 0,05 |
| Alanine sur Σ AAT | (g/g) | 0,08 | 0,06 |
| Valine sur Σ AAT | (g/g) | 0,06 | 0,05 |
| Isoleucine sur Σ AAT | (g/g) | 0,04 | 0,03 |
| Leucine sur Σ AAT | (g/g) | 0,08 | 0,06 |
| Tyrosine sur Σ AAT | (g/g) | 0,04 | 0,03 |
| Phénylalanine sur Σ AAT | (g/g) | 0,04 | 0,03 |
| Lysine sur Σ AAT | (g/g) | 0,06 | 0,05 |
| Histidine sur Σ AAT | (g/g) | 0,02 | 0,02 |
| **Arginine sur Σ AAT** | **(g/g)** | 0,06 | **0,10** |
| Proline sur Σ AAT | (g/g) | **0,04** | **0,07** |
| Cystine sur Σ AAT | (g/g) | 0,02 | 0,01 |
| Méthionine sur Σ AAT | (g/g) | 0,02 | 0,02 |
| Tryptophane sur Σ AAT | (g/g) | 0,02 | 0,01 |

Pour la souche *Schizochytrium sp* les effets sont identiques mais de moindre amplitude. Par ailleurs, on remarque aussi une augmentation de 75 % de la proportion de proline parmi les acides aminés.

Les teneurs en acides aminés arginine et acide glutamique augmentent respectivement de 60 et 75 % alors que la teneur en protéines double.

La teneur en DHA dans les acides gras augmente de 23%.

## Revendications

1. Procédé d'enrichissement d'une biomasse de microalgues du genre *Thraustochytrium* en acide docosahexaénoïque (DHA) et en acides aminés arginine et acide glutamique, **caractérisé en ce qu'**il comprend une étape visant à limiter le taux de croissance de la microalgue tout en maintenant ou en introduisant en continu une source d'azote dans le milieu de fermentation dès que la valeur du rapport des taux de croissance µ/µmax des microalgues devient inférieure à 0,2..

2. Procédé selon la revendication 1, **caractérisé en ce que** les microalgues sont du genre *Schizochytrium sp ou Schizochytrium mangrovei.*

3. Procédé selon l'une ou l'autre des revendications précédentes, **caractérisé en ce que** les microalgues sont une souche sélectionnée parmi les souches CNCM I-4469 et CNCM I-4702 déposées à la Collection Nationale de Cultures de Microorganismes de l'Institut Pasteur respectivement le 14 avril 2011 et le 22 novembre 2012.

4. Procédé selon l'une quelconque des revendications précédentes, **caractérisé en ce que** la limitation du taux de croissance de la microalgue est obtenue par la réduction ou l'épuisement en oligoéléments du milieu de fermentation ou par la limitation du transfert en O₂, de préférence par la limitation du transfert en O₂.

5. Procédé selon l'une quelconque des revendications précédentes, **caractérisé en ce qu'**il comprend en outre la récolte de la biomasse, éventuellement la préparation d'un extrait ou lysat cellulaire à partir de cette biomasse, puis facultativement l'extraction d'une huile brute riche en DHA.

6. Procédé selon l'une quelconque des revendications précédentes, **caractérisé en ce que** la biomasse obtenue comprend au minimum 45 % de DHA en poids d'acides gras totaux.

7. Procédé selon l'une quelconque des revendications précédentes, **caractérisé en ce que** la biomasse obtenue comprend au minimum 40 % de protéines en poids de biomasse (g/g) exprimées en N.6,25 dont au minimum 10% d'arginine et au minimum 25 % d'acide glutamique en poids sur acides aminés totaux.

## Patentansprüche

1. Verfahren zur Anreicherung einer Mikroalgenbiomasse der Gattung *Thraustochytrium* an Docosahexaensäure (DHA) und an den Aminosäuren Arginin und Glutaminsäure, **dadurch gekennzeichnet, dass** es einen Schritt umfasst, der darauf abzielt die Wachstumsrate der Mikroalgen zu begrenzen, unter Beibehaltung oder unter kontinuierlicher Zufuhr einer Stickstoffquelle in das Fermentationsmedium sobald der Wert des Verhältnisses der Wachstumsraten der Mikroalgen kleiner wird als 0,2.

2. Verfahren nach Anspruch 1, **dadurch gekennzeichnet, dass** die Mikroalgen von der Gattung *Schizochytrium sp* oder *Schizochytrium mangrovei* sind.

3. Verfahren nach einem der vorhergehenden Ansprüchen, **dadurch gekennzeichnet, dass** die Mikroalgen ein Stamm sind, der ausgewählt ist aus den Stämmen CNCM I-4469 und CNCM I-4702 die bei der Nationalen Sammlung von Mikroorganismenkulturen von Institut Pasteur am 14. April 2011 bzw. am 22. November 2012 hinterlegt worden sind.

4. Verfahren nach einem der vorhergehenden Ansprüche, **dadurch gekennzeichnet, dass** die Begrenzung der Wachstumsrate der Mikroalge durch die Reduktion oder die Verarmung des Fermentationsmediums an Mikronährstoffen oder durch die Begrenzung der Sauerstoffübertragung, vorzugsweise durch Begrenzung der Sauerstoffübertragung erhalten wird.

5. Verfahren nach einem der vorhergehenden Ansprüche, **dadurch gekennzeichnet, dass** es weiterhin das Ernten der Biomasse, gegebenenfalls die Herstellung eines Extraktes oder Zelllysats aus dieser Biomasse, dann eventuell die Extraktion eines Rohöl, das reich an DHA ist, umfasst.

6. Verfahren nach einem der vorhergehenden Ansprüche, **dadurch gekennzeichnet, dass** die erhaltene Biomasse mindestens 45 % DHA umfasst, bezogen auf das Gewicht der Fettsäuren insgesamt.

7. Verfahren nach einem der vorhergehenden Ansprüche, **dadurch gekennzeichnet, dass** die erhaltene Biomasse mindestens 40 % Proteine umfasst, bezogen auf das Gewicht der Biomasse (g/g), ausgedrückt in N.6,25, von denen mindestens 10% Arginin und mindestens 25% Glutaminsäure sind, bezogen auf das Gewicht der Aminosäuren insgesamt.

## Claims

1. A process for enriching a biomass of microalgae of the *Thraustochytrium* genus with docosahexaenoic acid (DHA) and with arginine and glutamic acid amino acids, **characterized in that** it comprises a step aimed at limiting the growth rate of the microalga while at the same time maintaining or continuously introducing a nitrogen source in or into the fermentation medium as soon as the value of the ratio of the growth rates µ/µmax of the microalgae becomes less than 0,2.

2. The process as claimed in claim 1, **characterized in that** the microalgae are of the genus *Schizochytrium sp.* or *Schizochytrium mangrovei* genus.

3. The process as claimed in either of the preceding claims, **characterized in that** the microalgae are a strain selected from strain references CNCM 1-4469 and CNCM 1-4702 deposited with the Collection Nationale de Cultures de Microorganismes of the Institut Pasteur on April 14, 2011 and November 22, 2012, respectively.

4. The process as claimed in any one of the preceding claims, **characterized in that** the limitation of the growth rate of the microalga is obtained by reducing or exhausting trace elements in the fermentation medium or by limiting the O₂ transfer, preferably by limiting the O₂ transfer.

5. The process as claimed in any one of the preceding claims, **characterized in that** it further comprises harvesting the biomass, optionally preparing a cell extract or lysate from this biomass, then optionally extracting a DHA-rich crude oil.

6. The process as claimed in any one of the preceding claims, **characterized in that** the biomass obtained comprises at least 45% of DHA by weight of total fatty acids.

7. The process as claimed in any one of the preceding claims, **characterized in that** the biomass obtained comprises at least 40% of proteins by weight of biomass (g/g) expressed in N.6,25, including at least 10% of arginine and at least 25% of glutamic acid by weight relative to total amino acids.


### SEQUENCE LISTING

<110> ROQUETTE FRERES
<120> PROCEDE D'ENRICHISSEMENT DE LA BIOMASSE DE MICROALGUES DU GENRE TRAUSTOCHYTRIUM EN DHA ET EN ACIDES AMINES ARG ET GLU
<130> B1954PC
<160> 2
<170> PatentIn version 3.5
<210> 1
   <211> 479
   <212> DNA
   <213> Schizochytrium sp
<400> 1
   gagggtttta cattgctctc attccaatag caagacgcga agcgccccgc attgatattt        60
   ctcgtcacta cctcgtggag tccacattgg gtaatttacg cgcctgctgc cttccttgga       120
   tgtggtagcc gtctctcagg ctccctctcc ggagtcgagc cctaactccc cgtcacccgt       180
   tatagtcacc gtaggccaat accctaccgt cgacaactga tggggcagaa actcaaacga       240
   ttcatcgctc cgaaaagcga tctgctcaat tatcatgact caccaagaga gttggcttag       300
   acctaataag tgcggccctc cccgaaagtc gggcccgtac agcacgtatt aattccagaa       360
   ttactgcagg tatccgtata aaggaactac cgaagggatt ataactgata taatgagccg       420
   ttcgcagttt cacagtataa ttcgcttata cttacacatg catggcttag tctttgaga        479
<210> 2
   <211> 454
   <212> DNA
   <213> Schizochytrium mangrovei
<400> 2
   ggttttacat tgctctcatt ccgatagcaa aacgcataca cgcttcgcat cgatatttct      60
   cgtcctacct cgtggagtcc acagtgggta atttacgcgc ctgctgctat ccttggatat     120
   ggtagccgtc tctcaggctc cctctccgga gtcgagccct aactctccgt cacccgttat     180
   agtcaccgta gtccaataca ctaccgtcga caactgatgg ggcagaaact caaacgattc     240
   atcgaccaaa awagtcaatc tgctcaatta tcatgattca ccaataaaat cggcttcaat     300
   ctaataagtg cagccccata cagggctctt acagcatgta ttatttccag aattactgca     360
   ggtatccata taaaagaaac taccgaagaa attattactg atataatgag ccgttcgcag     420
   tctcacagta caatcgctta tacttacaca gcag                                 454